# EUROPÄISCHE PATENTSCHRIFT

(11) **EP 1 999 460 B1**
(45) Veröffentlichungstag und Bekanntmachung des Hinweises auf die Patenterteilung: **24.03.2010**
(21) Anmeldenummer: 07703042.7
(22) Anmeldetag: 25.01.2007
(51) Int. Cl.: G01N 25/58, G01N 21/55

(54) **VERFAHREN UND SENSOR ZUM BESTIMMEN DES KOHLENWASSERSTOFF-TAUPUNKTES IN EINEM GAS**
METHOD AND SENSOR FOR DETERMINING THE HYDROCARBON DEW POINT IN A GAS
PROCÉDÉ ET CAPTEUR POUR DÉTERMINER LE POINT DE ROSÉE DE HYDROCARBURE DANS UN GAZ

(30) Priorität: 24.03.2006 DE 102006013726
(43) Veröffentlichungstag der Anmeldung: 10.12.2008
(73) Patentinhaber: Bartec GmbH, 97980 Bad Mergentheim (DE)
(72) Erfinder: BÖHM, Alfred, 94234 Viechtach (DE)
(74) Vertreter: Heim, Hans-Karl
(86) Internationale Anmeldenummer: PCT/EP2007/000652
(87) Internationale Veröffentlichungsnummer: WO 2007/110118

(56) Entgegenhaltungen:
- WO-A-2006/015734
- DE-A1- 3 543 155
- US-A- 4 946 288

## Beschreibung

Die Erfindung betrifft ein Verfahren und einen Sensor zum Bestimmen des Kohlenwasserstoff-Taupunktes in einem Gas, insbesondere in einem gasförmigen Brennstoff.

Bei der Kohlenwasserstoff-Taupunkttemperatur handelt es sich um eine bedeutende Messgröße zur Charakterisierung der Qualität von Erdgas oder anderen gasförmigen Brennstoffen.

Eine Vorrichtung und ein Verfahren zur Bestimmung des Kohlenwasserstoff-Taupunktes in einem Gas sind aus der US 4,946,288 bekannt. Diese Druckschrift lehrt, eine Betauungsfläche durch ein Messgasvolumen hindurch mit Licht zu bescheinen. Von der Betauungsfläche gestreutes Licht wird, nachdem es abermals das Messgas durchlaufen hat, mittels einer CCD-Anordnung nachgewiesen. Die Betauungsfläche wird abgekühlt und das Erreichen der Taupunkttemperatur kann dabei als Abnahme der Streulichtintensität nachgewiesen werden.

Bei der aus der US 4,946,288 bekannten Vorrichtung wird das Licht mehrfach durch das Messgas geführt. Dies macht eine vergleichsweise voluminöse Messkammer erforderlich. Daneben ist die bekannte Vorrichtung auch vergleichsweise aufwändig in der Kalibrierung und aufgrund des direkten Kontaktes einer Reihe von Komponenten mit dem Messgas vergleichsweise verschmutzungsempfindlich. Hierdurch ist die bekannte Vorrichtung vergleichsweise kostenaufwändig.

Zur Bestimmung der Kohlenwasserstoff-Taupunkttemperatur ist aus der Firmenschrift "Condumax - Taupunktanalysator für Kohlenwasserstoffe" der Firma Michell Instruments ferner das so genannte "Dark-Spot"-Prinzip bekannt. Hierbei wird ein gebündelter Lichtstrahl auf eine Oberfläche mit einer konischen Vertiefung fokussiert und die Temperatur der Oberfläche variiert. Im unbetauten Zustand wird ein Großteil des Lichtes als ringförmige Reflexion abgebildet. Das Streulicht innerhalb dieses Lichtringes wird optisch erfasst. Wenn sich Kondensat auf der Oberfläche niederschlägt, ändern sich die optischen Verhältnisse und die Intensität des reflektierten Lichtes im Bereich des Ringes erhöht sich, während sich die Streulichtintensität im Bereich des so genannten "Dark-Spot" reduziert. Diese Effekte können zum Nachweis der Betauung erfasst werden. Auch beim "Dark-Spot"-Verfahren wird das Licht durch das Messgasvolumen hindurchgestrahlt, was die oben genannten unerwünschten Folgen haben kann.

Aus der DE 35 43 155 ist ein optischer Taupunktsensor mit einer Lichtleitfaser bekannt, die eine gezielte Beschädigung aufweist. Eine solche Lichtleitfaser ist im Betrieb jedoch vergleichsweise beschädigungsanfällig. Ferner kann es schwierig sein, die erwünschte Beschädigung der Faser bei der Herstellung mit hinreichender Genauigkeit zu reproduzieren, was einen hohen Kalibrierungsaufwand mit sich bringen kann.

Ein weiterer Taupunktsensor, der zum Bestimmen der Temperatur von Atmosphärenwasser vorgesehen ist, ist in der US 3,528,278 beschrieben. Bei dieser Vorrichtung wird das Licht durch ein kristallines Prisma hindurch zu Kondensationsbereichen geführt, die auf einer planaren Oberfläche des Prismas angeordnet sind, und die mit dem Messgas in Kontakt stehen. Das von den Kondensationsbereichen in das Prisma zurückreflektierte Licht wird mit einem Lichtsensor gemessen. Schlägt sich in den Kondensationsbereichen ein Wasserkondensat nieder, so ändert sich dort der Grenzwinkel für die Totalreflexion des Lichtes. Hieraus folgt beim Einsatz von Betauung eine Lichtauskopplung aus dem Prisma, die am Lichtsensor als Abnahme der Lichtintensität nachgewiesen werden kann.

Wird der bekannte Taupunktsensor jedoch für Kohlenwasserstoffe eingesetzt, so stellt sich bei Unterschreiten des Taupunktes häufig nur eine geringe Abnahme der reflektierten Lichtintensität ein, so dass Messfehler resultieren können oder sich die Taupunktbestimmung sogar als völlig unmöglich gestalten kann.

Ein ähnlich arbeitender Taupunktsensor für Atmosphärenwasser ist in der deutschen Patentanmeldung DE 10 2004 038 397.9 beschrieben. Dieser Taupunktsensor weist eine planare Kondensationsoberfläche mit semihydrophoben Eigenschaften auf. Aufgrund der semihydrophoben Oberflächeneigenschaften bilden kondensierende Wassertröpfchen eine spezifische Form, die eine besonders wirksame Lichtauskopplung bei Betauung gewährleistet. Somit wird ein besonders ausgeprägter Intensitätsabfall in der reflektierten Lichtintensität beim Abkühlen unter den Taupunkt und folglich eine besonders zuverlässige Taupunktbestimmung gewährleistet.

Beim Einsatz dieser Sensoren für Kohlenwasserstoffe tritt bei Unterschreiten des Taupunktes jedoch häufig ebenfalls nur eine geringe Abnahme der reflektierten Lichtintensität auf, was die Taupunktbestimmung erschweren kann.

**Aufgabe** der Erfindung ist es, ein Verfahren und einen Sensor zum Bestimmen des Kohlenwasserstoff-Taupunktes anzugeben, die bei kompakten Sensorabmessungen besonders einfach, wirtschaftlich und zuverlässig sind.

Die Aufgabe wird erfindungsgemäß durch ein Verfahren mit den Merkmalen des Anspruchs 1, und einen Sensor mit den Merkmalen des Anspruchs 2 gelöst. Bevorzugte Ausführungsbeispiele sind in den jeweils abhängigen Ansprüchen angegeben.

Die Erfindung lehrt ein Verfahren zum Bestimmen des Kohlenwasserstoff-Taupunktes in einem Gas, insbesondere in einem gasförmigen Brennstoff, mittels eines Sensors mit einem transparenten Körper, der eine planare Messoberfläche aufweist, auf der eine aufgeraute Kondensationsoberfläche mit kondensationsabhängigem Reflexionsvermögen vorgesehen ist, bei dem die Kondensationsoberfläche mit dem Gas beaufschlagt wird, die Kondensationsoberfläche durch den transparenten Körper hindurch mit Licht bestrahlt wird, die Temperatur der Kondensationsoberfläche verändert wird, die von der Kondensationsoberfläche in den transparenten Körper zurückreflektierte Lichtintensität bestimmt wird, und beim Auftreten einer Intensitätszunahme des zurückreflektierten Lichtes bei abnehmender Temperatur und/oder beim Auftreten einer Intensitätsabnahme.des zurückreflektierten Lichtes bei steigender Temperatur der Kondensationsoberfläche die aktuelle Temperatur der Kondensationsoberfläche bestimmt wird und als Maß für die Taupunkttemperatur ausgegeben wird.

Ferner lehrt die Erfindung einen Sensor zum Bestimmen des Kohlenwasserstoff-Taupunktes in einem Gas, bevorzugt in einem gasförmigen Brennstoff, mit einem transparenten Körper mit einer planaren Messoberfläche, auf der eine aufgeraute Kondensationsoberfläche angeordnet ist, einer Lichtquelle zum Aussenden von Licht durch den transparenten Körper auf die Kondensationsoberfläche, einem Lichtdetektor zum Ermitteln der von der Kondensationsoberfläche in den transparenten Körper zurückreflektierten Lichtintensität, Mitteln zum Einstellen der Temperatur der Kondensationsoberfläche, und einer Auswerteeinheit, die mit dem Lichtdetektor in Signalverbindung steht, und die dazu eingerichtet ist, beim Auftreten einer Intensitätszunahme des zurückreflektierten Lichtes bei abnehmender Temperatur und/oder einer Intensitätsabnahme des zurückreflektierten Lichtes bei steigender Temperatur der Kondensationsoberfläche die entsprechende Temperatur der Kondensationsoberfläche zu bestimmen und als Maß für die Taupunkttemperatur auszugeben.

Der erfindungsgemäße Sensor kann insbesondere zur Durchführung des erfindungsgemäßen Verfahrens verwendet werden, wodurch sich die in diesem Zusammenhang diskutierten Vorteile erzielen lassen. Die im Zusammenhang mit dem Sensor diskutierten Ausführungsbeispiele können auch beim erfindungsgemäßen Verfahren zum Einsatz kommen. Ebenso können die im Zusammenhang mit dem Verfahren diskutieren Ausführungsbeispiele beim erfindungsgemäßen Sensor eingesetzt werden.

Nach der Erfindung ist ein optischer Taupunktsensor vorgesehen, bei dem die optische Detektionsstrecke im Bereich der Kondensationsoberfläche nicht durch das Messgas hindurch verläuft. Vielmehr wird die Kondensationsoberfläche erfindungsgemäß durch den transparenten Körper hindurch, sozusagen "rückseitig" beschienen. Verglichen mit den nach dem Stand der Technik zum Bestimmen des Kohlenwasserstoff-Taupunktes vorgesehenen Sensoren ist somit keine voluminöse Messkammer erforderlich und es ist ein besonders kompakter und verschmutzungsunempfindlicher, also zuverlässiger Sensor gegeben.

Ein weiterer Aspekt der Erfindung kann darin gesehen werden, dass die rückseitig beschienene Kondensationsoberfläche des Sensors nicht mikroskopisch glatt sondern aufgeraut ausgebildet ist, und dass eine Intensitätszunahme des von dieser rauen Kondensationsoberfläche in den transparenten Körper zurückreflektierten Lichtes, nicht hingegen eine Intensitätsabnahme, als Detektionskriterium für das Unterschreiten der Taupunkttemperatur herangezogen wird.

Diesem Erfindungsaspekt liegt die Erkenntnis zugrunde, dass die relevanten Kohlenwasserstoffe beim Unterschreiten des Taupunktes auf den vorbekannten Kondensationsoberflächen häufig kleine Kontaktwinkel ausbilden oder sogar spreiten. Ursache hierfür ist, dass die Oberflächenerergien der relevanten Kohlenwasserstoffe in der Regel deutlich kleiner als die Oberflächenenergien der bekannten Kondensationsoberflächen sind. Beim Unterschreiten der Taupunkttemperatur an der Kondensationsoberfläche bildet das Kohlenwasserstoffkondensat somit keine kugelartigen Tröpfchen, sondern einen im Wesentlichen glatten Kondensatfilm mit sehr flachen Randwinkeln, dessen Oberfläche am Übergang zum Messgas in großen Bereichen etwa parallel zur Kondensationsoberfläche verläuft.

Wird in diesem Fall, wie aus dem Stand der Technik bekannt, eine glatte Kondensationsoberfläche verwendet, so hat diese Filmbildung des Kondensats zur Folge, dass sich die Intensität des in den transparenten Körper zurückreflektierten Lichtes beim Unterschreiten des Taupunktes allenfalls geringfügig ändert und somit ein sicherer Taupunktnachweis nicht möglich ist. Dies liegt darin begründet, dass bei einsetzender Betauung zwar zunächst ein größerer Anteil von Lichtintensität aus dem transparenten Körper ausgekoppelt wird als bei trockener Oberfläche. Die ausgekoppelte Lichtintensität wird aber an der Grenzschicht Kondensat-Gas, die parallel zur glatten Oberfläche verläuft, wieder reflektiert und gelangt von dort wieder zurück in den transparenten Körper und von dort zum Fotodetektor. Ein Sensor mit glatter Kondensationsoberfläche, bei dem ein Intensitätsabfall der reflektierten Lichtintensität als Kriterium für die einsetzende Betauung herangezogen wird, ist somit zur Taupunktbestimmung von Kohlenwasserstoffen häufig nicht geeignet.

Die Erfindung beschreitet demgegenüber einen anderen Weg. Einerseits wird keine glatte sondern eine aufgeraute Kondensationsoberfläche verwendet, andererseits wird nicht eine Abnahme sondern eine Zunahme der reflektierten Lichtintensität als Nachweiskriterium für die einsetzende Betauung verwendet. Die Aufrauung der Kondensationsoberfläche hat zur Folge, dass ein großer Teil aller auf die Kondensationsoberfläche eingestrahlten Lichtstrahlen mikroskopisch gesehen unter einem Einfallswinkel einfällt, der kleiner als der Grenzwinkel für Totalreflexion ist. Aufgrund der Oberflächengestaltung tritt also im trockenen Zustand ein signifikanter Teil der einfallenden Lichtintensität an der Kondensationsoberfläche aus dem transparenten Körper aus und wird nicht in diesen zurückreflektiert. Die Oberflächenaufrauung bildet sozusagen "künstliche Tröpfchen" oder "künstliche Eiskristalle", die bei trockener Oberfläche für eine erhebliche Lichtauskopplung sorgen.

Die Verhältnisse ändern sich, wenn die raue Oberfläche bei Unterschreiten des Taupunktes benetzt wird. In diesem Fall füllen sich die Unebenheiten auf der Kondensationsoberfläche mit Kondensat auf. Infolgedessen wird ein Teil der Lichtintensität, die aus dem transparenten Körper austritt, an der Grenzfläche Kondensat-Gas in den transparenten Körper zurückreflektiert. Beim Einsetzen von Betauung der Kondensationsoberfläche nimmt somit die in den Lichtleiter zurückreflektierte Lichtintensität zu, was erfindungsgemäß als Kriterium für das Unterschreiten des Taupunktes herangezogen wird.

Es hat sich gezeigt, dass die Änderung der Lichtintensität bei dem erfindungsgemäßen Sensor besonders groß ist, wenn das Kondensat auf der Oberfläche spreitet. Kleine Kontaktwinkel, die bei den bekannten Sensoren nachteilig sein können, können somit nach der Erfindung besonders erwünscht sein. Verglichen mit vorbekannten Sensoren mit glatter Kondensationsoberfläche weist der erfindungsgemäße Sensor ein invertiertes Ansprechverhalten auf. Durch abwechselndes Heizen und Kühlen der Kondensationsoberfläche auf Temperaturen oberhalb beziehungsweise unterhalb des Taupunktes kann nach der Erfindung laufend der Kohlenwasserstoff-Taupunkt ermittelt werden.

Ein weiterer Aspekt der Erfindung kann darin gesehen werden, dass die Kondensationsoberfläche auf einer planaren Messoberfläche vorgesehen ist. Auf einer solchen planaren Messoberfläche kann die Aufrauung der Kondensationsoberfläche in besonders einfacher Weise und mit hoher Reproduzierbarkeit erstellt werden. Der erfindungsgemäße Sensor kann somit auch in großen Stückzahlen bei niedrigem Kalibrierungsaufwand besonders einfach und wirtschaftlich gefertigt werden.

Erfindungsgemäß sind insbesondere die Anforderungen hinsichtlich der Justierung der Lichtquelle und des Lichtdetektors vergleichsweise gering, da bei einer aufgerauten planaren Kondensationsoberfläche ein Einsetzen von Betauung auch bei nichtsymmetrischer Anordnung von Lichtquelle und Lichtdetektor bezüglich der Kondensationsoberfläche angezeigt wird.

Die Erfindung ist besonders geeignet zum Bestimmen des Kohlenwasserstoff-Taupunktes in einem gasförmigen Brennstoff, beispielsweise in Erdgas oder Biogas. Unter dem Kohlenwasserstoff-Taupunkt kann insbesondere die Temperatur verstanden werden, bei deren Unterschreitung Kohlenwasserstoffe aus dem Gas auskondensieren oder ausfrieren.

Soweit nach der Erfindung vorgesehen ist, die Temperatur der Kondensationsoberfläche auszugeben, kann dies beispielsweise eine direkte Ausgabe auf eine Anzeigeeinrichtung beinhalten. Die Ausgabe kann aber auch beispielsweise an eine Speichereinrichtung erfolgen, die vorzugsweise für eine zeitlich verzögerte Darstellung der Temperatur und/oder für eine weitere Datenverarbeitung vorgesehen sein kann. Da die Intensitätsänderung des zurückreflektierten Lichtes, insbesondere aufgrund kinetischer Effekte, in der Regel kein sprunghafter sondern ein kontinuierlicher Vorgang ist, kann vorgesehen sein, die bei der Lichtintensitätsänderung erfasste Oberflächentemperatur nicht unmittelbar mit der Taupunkttemperatur gleichzusetzen, sondern diese mittels geeigneter Rechenanweisungen zu korrigieren. Außer bei der angegebenen Bedingung kann eine Temperaturbestimmung auch zu anderen Zeitpunkten erfolgen, beispielsweise zur Überwachung der Sensorfunktion. Insbesondere kann eine kontinuierliche Temperaturbestimmung vorgesehen sein, wobei jedoch nur die bei der genannten Bedingung vorliegenden Temperaturwerte als Taupunkt ausgegeben werden. Die Auswerteeinheit kann beispielsweise als Rechner ausgebildet sein.

Eine besonders genaue Taupunktbestimmung wird erfindungsgemäß dadurch ermöglicht, dass eine Einrichtung zum Messen der Temperatur der Kondensationsoberfläche vorgesehen ist, die mit der Auswerteeinheit in Signalverbindung steht. Diese Einrichtung kann beispielsweise einen temperaturabhängigen Leiter aufweisen, der bevorzugt Platin aufweist. Der temperaturabhängige Leiter kann beispielsweise als strukturierte Schicht ausgebildet sein, die auf der Messoberfläche angeordnet ist. Geeigneterweise umgibt der temperaturabhängige Leiter die Kondensationsoberfläche U-förmig. Es kann aber auch grundsätzlich vorgesehen sein, die Temperatur indirekt, beispielsweise durch Erfassung der Betriebsparameter der Mittel zum Einstellen der Temperatur der Kondensationsoberfläche, zu erfassen.

Ein besonders robuster und zugleich wirtschaftlicher Sensor ist dadurch gegeben, dass der transparente Körper ein Glaskörper, insbesondere ein Borosilikat-Glaskörper, bevorzugt ein Borofloat-Glaskörper ist. Der transparente Körper kann beispielsweise auch Kalk-Natronglas aufweisen. Auch kann ein Plexiglas aufweisender Körper vorgesehen werden. Erfindungsgemäß ist der Körper im Wellenlängenbereich der Lichtquelle, der insbesondere im VIS- oder IR-Bereich, beispielsweise im NIR-Bereich, liegen kann, transparent.

Vorteilhaft ist es nach der Erfindung, dass die aufgeraute Kondensationsoberfläche eine Glasoberfläche oder eine beschichtete Glasoberfläche aufweist.

Vorzugsweise ist auf der Messoberfläche, insbesondere auf einem Temperatursensor, eine Passivierungsschicht vorgesehen, die dazu beitragen kann, Kurzschlüsse zu vermeiden. Eine solche Passivierungsschicht kann beispielsweise SiOₓ, SiONₓ oder SiC aufweisen.

Insbesondere ist es bevorzugt, dass die Kondensationsoberfläche eine Wasser und/oder Schmutz abweisende Beschichtung aufweist. Hierbei kann es sich insbesondere um eine hydrophobe Schicht, bevorzugt um eine ultrahydrophobe Schicht, beispielsweise mit Wasserkontaktwinkeln größer als 120°, handeln. Beispielsweise kann eine PTFE-Schicht (Teflonschicht) und/oder eine nanotechnologische, insbesondere nanoraue, Schicht vorgesehen sein. Eine Wasser abweisende Beschichtung kann einen "Lotus-Effekt" erzeugen, der den Sensor besonders verschmutzungsunempfindlich macht. Außer auf der Kondensationsoberfläche kann eine Wasser und/oder Schmutz abweisende Beschichtung auch auf den die aufgeraute Kondensationsoberfläche umgebenden Oberflächenbereichen der Messoberfläche vorgesehen sein.

Außerhalb der Kondensationsoberfläche, insbesondere auf seiner der Kondensationsoberfläche abgewandten Seite, ist der transparente Körper bevorzugt verspiegelt, beispielsweise mittels einer NiCr-Schicht.

Besonders vorteilhaft ist es nach der Erfindung, dass die Kondensationsoberfläche lipophil ausgebildet ist. Hierunter kann verstanden werden, dass die nachzuweisenden Kohlenwasserstoffe auf der Kondensationsoberfläche kleine Kontaktwinkel, insbesondere Randwinkel von weniger als 90°, ausbilden. Besonders bevorzugt ist es jedoch, dass die Kontaktwinkel kleiner als 25°, insbesondere kleiner als 10° sind.

Um unerwünschte Beugungseffekte zu vermeiden, ist es vorteilhaft, wenn die gemittelte Rautiefe der Kondensationsoberfläche zumindest eine Größenordnung größer als die Wellenlänge des verwendeten Lichtes ist. Um einen sicheren Nachweis der Betauung zu gewährleisten, ist es ferner vorteilhaft, wenn die gemittelte Rautiefe der Kondensationsoberfläche zumindest eine Größenordnung kleiner als die laterale Abmessung der Kondensationsoberfläche ist. Besonders bevorzugt ist es, dass die Kondensationsoberfläche eine gemittelte Rautiefe zwischen 1 µm und 100 µm, insbesondere von etwa 10 µm, aufweist.

Die Aufrauung der Kondensationsoberfläche kann durch Zufügen von Material auf die Messoberfläche gebildet sein. Insbesondere in diesem Fall kann die Kondensationsoberfläche gegenüber der umliegenden Messoberfläche im Mittel eine Erhöhung bilden. Die Aufrauung kann aber auch Material abtragend hergestellt sein. Insbesondere in diesem Fall kann die Kondensationsoberfläche im Mittel tiefer liegen als die umgebende Messoberfläche, das heißt die Kondensationsoberfläche kann in den transparenten Körper hinein versetzt sein. Auch gemischte Herstellungsweisen sind denkbar, bei denen zunächst zusätzlich Material aufgebracht wird und dieses Material zur Vergrößerung der Rauigkeit wieder abgetragen wird.

Die Rauigkeit der Kondensationsoberfläche kann statistisch verteilt sein, das heißt die Oberflächenrauigkeit kann durch einen Zufallsprozess, beispielsweise Sandstrahlen, gebildet sein. In diesem Fall weist die Kondensationsoberfläche eine unregelmäßige Topographie auf. Die Kondensationsoberfläche kann aber auch strukturiert sein, das heißt zum Herstellen der Rauigkeit wird der Oberfläche eine vorher bestimmte Topographie aufgeprägt. Dabei kann die Oberflächentopographie regelmäßig sein. In diesem Fall können beispielsweise erhabene oder vertiefte Pyramiden und/oder konvexe oder konkave Rillen auf der Oberfläche vorgesehen sein.

Die Messgenauigkeit kann weiter dadurch erhöht werden, dass die Messoberfläche in der Umgebung der Kondensationsoberfläche ebenfalls aufgeraut ist. Die Kondensationsoberfläche kann grundsätzlich die gesamte planare Messoberfläche einnehmen.

Ein konstruktiv besonders einfacher und zugleich zuverlässiger Sensor ist dadurch gegeben, dass der transparente Körper eine der planaren Messoberfläche abgewandte Rückseitenfläche aufweist, die zumindest bereichsweise parallel zur Messoberfläche verläuft, wobei die Lichtquelle und/oder der Lichtdetektor in den parallel verlaufenden Bereichen der Rückseitenfläche angeordnet sind.

Besonders bevorzugt ist es ferner, dass die Lichtquelle und/oder der Lichtdetektor bezüglich der Kondensationsoberfläche seitlich versetzt angeordnet sind. Gemäß dieser Ausführungsform wird die Kondensationsoberfläche schräg mit Licht bestrahlt und/oder das schräg zurückreflektierte Licht nachgewiesen. Grundsätzlich ist es aber auch möglich, die Lichtquelle und/oder den Lichtdetektor auf einer durch die Kondensationsoberfläche verlaufenden Senkrechten anzuordnen. Geeigneterweise sind die Lichtquelle und der Lichtdetektor symmetrisch bezüglich der Kondensationsoberfläche angeordnet, das heißt der Einfallswinkel, unter dem Licht auf die Kondensationsoberfläche eingestrahlt wird, entspricht dem Ausfallswinkel, unter dem zurückreflektiertes Licht nachgewiesen wird. Da erfindungsgemäß eine aufgeraute Kondensationsoberfläche vorgesehen ist, ist jedoch auch eine asymmetrische Anordnung von Lichtquelle und Lichtdetektor denkbar, bei der sich der Einfallswinkel des Lichtes vom Ausfallswinkel, unter dem nachgewiesen wird, unterscheidet.

Eine besonders einfache, schnelle und genaue Temperaturkontrolle der Kondensationsoberfläche kann dadurch gewährleistet werden, dass die Mittel zum Einstellen der Temperatur der Kondensationsoberfläche ein Peltier-Element aufweisen. Da Kohlenwasserstoff-Taupunkte in üblichen gasförmigen Brennstoffen unter üblichen Bedingungen häufig vergleichsweise tief unterhalb der Raumtemperatur liegen, kann es vorteilhaft sein, ein mehrstufiges Peltier-Element zu verwenden, das mehrere in Serie angeordnete Einzel-Peltier-Elemente aufweisen kann. Zum Erzeugen besonders tiefer Temperaturen kann auch vorgesehen sein, das Peltier-Element mittels eines Kühlmittels, beispielsweise flüssigem Stickstoff oder gefrorenem Kohlendioxid, vorzukühlen. In diesem Fall kann am Peltier-Element eine Kühlmittelaufnahme vorgesehen sein.

Erfindungsgemäß kann sowohl das Unterschreiten als auch das Überschreiten der Taupunkttemperatur an der Kondensationsoberfläche durch eine Änderung der Lichtintensität nachgewiesen werden. Besonders bevorzugt ist es, dass die Taupunktbestimmung jedenfalls bei sinkender Kondensationsoberflächentemperatur durchgeführt wird. Es kann aber auch ein Messmodus vorgesehen sein, bei dem die Temperaturbestimmung sowohl bei sinkender als auch bei steigender Temperatur vorgesehen ist, wobei die aus den unterschiedlichen Temperaturverläufen resultierenden Werte dann beispielsweise gemittelt werden können.

Nach der Erfindung ist es ferner vorteilhaft, dass eine Druckerzeugungseinrichtung vorgesehen ist, durch welche das Gas an der Kondensationsoberfläche mit Druck beaufschlagbar ist. Eine definierte Druckbeaufschlagung des Gases geht mit einer definierten Erhöhung des Taupunktes einher, so dass gemäß dieser Ausführungsform der Aufwand zur Kühlung der Kondensationsoberfläche verringert werden kann.

Erfindungsgemäß wird der Sensor zur Bestimmung der Taupunkttemperatur von Kohlenwasserstoffen, insbesondere von gasförmigen Brennstoffen wie Erdgas oder Biogas, eingesetzt.

Ein besonders einfaches Verfahren zum Herstellen eines erfindungsgemäßen Sensors ist **dadurch gekennzeichnet, dass** der transparente Körper im Bereich der Kondensationsoberfläche durch chemisches Ätzen, Strahlmattierung, Lasermattierung und/oder Beschichten aufgeraut wird. Insbesondere kann der transparente Körper vor dem Herstellen der Aufrauung maskiert werden, um eine gezielte Aufrauung der Kondensationsoberfläche ohne Beeinflussung der verbleibenden Oberflächenbereiche zu gewährleisten. In besonders einfacher Weise kann die Aufrauung der Kondensationsoberfläche in einem nasschemischen Ätzvorgang erzeugt werden. Zum chemischen Ätzen kann beispielsweise ein Ätzmittel verwendet werden, das Fluorverbindungen, insbesondere Flusssäure und/oder Mattiersalze mit Alkalifluoriden, beispielsweise Natriumfluorid oder Kaliumdifluorid, enthält. Es kann auch ein Ätzvorgang in Fluorwasserstoffdampf vorgesehen sein. Auch können Ammoniumhydrogendifluorid enthaltende Ätzmittel verwendet werden.

Zur Strahlmattierung kann vorgesehen sein, Strahlpartikel wie Quarzsand, Korund, Siliziumcarbid und/oder Glasgranulat unter Materialabtrag auf die Oberfläche zu leiten.

Die aufgeraute Kondensationsoberfläche kann aber auch durch Beschichtung erzeugt werden. Insbesondere kann eine glatte Oberfläche durch Aufbringen einer rauen Schicht aufgeraut werden, ohne dass die darunter liegende glatte Oberfläche selbst abgetragen wird. Zur Beschichtung der Kondensationsoberfläche kann beispielsweise ein elektrostatisches Beschichtungsverfahren vorgesehen werden. Demgemäß weist der erfindungsgemäße Sensor an der Kondensationsoberfläche geeigneterweise eine Beschichtung auf, deren Rauigkeit größer als die Rauigkeit der darunter liegenden Oberfläche des transparenten Körpers ist.

Die Erfindung wird nachfolgend anhand bevorzugter Ausführungsbeispiele näher erläutert, die in den Figuren dargestellt sind. In den Figuren zeigen schematisch:
- Fig. 1: eine Querschnittsansicht eines erfindungsgemäßen Sensors zur Durchführung des erfindungsgemäßen Ver- fahrens gemäß einer ersten Ausführungsform;
- Fig. 2: eine Draufsicht eines erfindungsgemäßen Sensors ge- mäß einer weiteren Ausführungsform;
- Fig. 3: ein beispielhaftes Oberflächenprofil der Messober- fläche eines erfindungsgemäßen Sensors im Bereich der aufgerauten Kondensationsoberfläche;
- Fig. 4: eine Querschnittsansicht eines weiteren Ausfüh- rungsbeispiels einer erfindungsgemäßen Kondensati- onsoberfläche in einer Messoberfläche;
- Fig. 5: eine vergrößerte Ansicht der Messoberfläche aus Fig. 4 bei Betauung;
- Fig. 6: eine Querschnittsansicht eines weiteren Ausfüh- rungsbeispiels einer erfindungsgemäßen Kondensati- onsoberfläche in einer Messoberfläche;
- Fig. 7: eine vergrößerte Ansicht der Messoberfläche aus Fig. 6 bei teilweiser Betauung;
- Fig. 8: eine Querschnittsansicht eines weiteren Ausfüh- rungsbeispiels einer erfindungsgemäßen Kondensati- onsoberfläche in einer Messoberfläche;
- Fig. 9: eine vergrößerte Ansicht der Messoberfläche aus Fig. 8 bei Betauung;
- Fig. 10: eine Draufsicht auf die Oberfläche der Fig. 8;
- Fig. 11: eine Draufsicht auf ein weiteres Ausführungsbei- spiel einer erfindungsgemäßen Kondensationsoberflä- che auf einer Messoberfläche; und
- Fig. 12: eine Draufsicht auf ein weiters Ausführungsbeispiel einer erfindungsgemäßen Kondensationsoberfläche auf einer Messoberfläche.

Gleichwirkende Elemente sind in den Figuren durchgehend mit denselben Bezugszeichen gekennzeichnet.

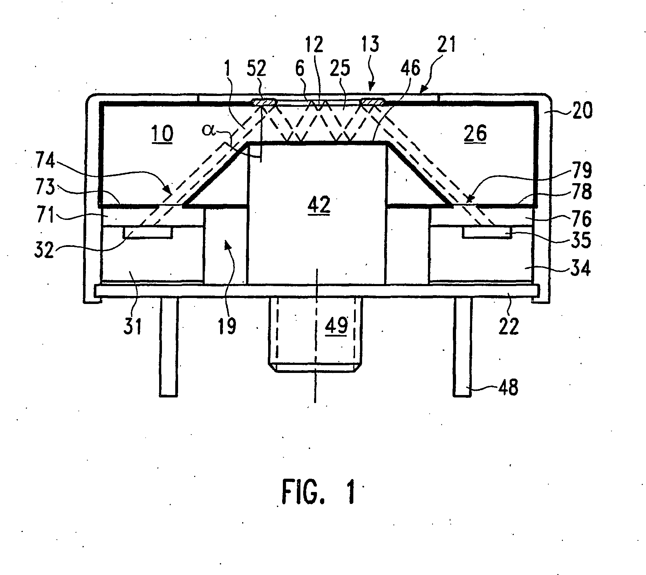

Ein erstes Ausführungsbeispiel eines erfindungsgemäßen Sensors ist in Fig. 1 dargestellt. Der Sensor weist einen primsmaartig und als Glassubstrat ausgebildeten, transparenten Körper 10 auf. Vorderseitig weist der transparente Körper 10 eine planare Messoberfläche 13 auf, die bei der Messung mit dem zu untersuchenden Gas in Kontakt gebracht wird. Auf der Rückseite 19 des transparenten Körpers 10 ist in einem parallel zur Messoberfläche 13 verlaufenden Bereich eine als Leuchtdiode ausgebildete Lichtquelle 31 angeordnet. Diese Lichtquelle 31 weist einen Sendechip 32 zum Aussenden von Licht im Nahinfrarotbereich auf. In diesem Spektralbereich ist auch der transparente Körper 10, der insbesondere aus einem Glas oder einem Kunststoff bestehen kann, lichtdurchlässig. Zwischen der als Leuchtdiode ausgebildeten Lichtquelle 31 und dem transparenten Körper 10 sind eine haftungsvermittelnde, transparente Immersionsschicht 71 sowie eine Reflexionsschicht 73 vorgesehen. Die Reflexionsschicht 73 ist auf den Lichtleiter 10 aufgedampft. Sie weist eine Ausnehmung 74 auf, durch welche eine Blende gebildet wird, durch die Lichtstrahlung der Lichtquelle 31 in Form eines Lichtbündels 1 in den transparenten Körper 10 tritt.

Mittig auf der vorderseitigen Messoberfläche 13 weist der transparente Körper 10 eine Kondensationsoberfläche 12 auf, deren Betauung im erfindungsgemäßen Verfahren nachweisbar ist. Die Kondensationsoberfläche 12 weist eine mikroskopische Rauigkeit mit einem Mittenrauwert von einigen 10 µm auf. Die mikroskopische Rauigkeit ist in der makroskopischen Darstellung der Fig. 1, die typischerweise Millimeterabmessungen bis Zentimeterabmessungen zeigt, nicht erkennbar. In der makroskopischen Ansicht ist die Kondensationsoberfläche 12 wie auch die umgebende, mikroskopisch glatte, Messoberfläche 13 planar.

Auf der Rückseite 19 des transparenten Körpers sind als Peltier-Element 42 ausgebildete Mittel zum Einstellen der Temperatur der Kondensationsoberfläche 12 angeordnet. Für eine besonders gute thermische Ankopplung der Kondensationsoberfläche 12 an das Peltier-Element 42 ist dieses über eine Wärme leitende Schicht, die beispielsweise Wärmeleitpaste aufweisen kann, am transparenten Körper 10 angeordnet.

Zur weiteren Verbesserung der Wärmekopplung der Kondensationsoberfläche 12 an das Peltier-Element 42 ist der Lichtleiter 10 im Bereich zwischen der Kondensationsoberfläche 12 und dem Peltier-Element 42 rückseitig mit einer Verjüngung 25 ausgebildet, in deren Bereich die Stärke des transparenten Körpers 10 auf etwa 1 mm verringert ist. Die vorderseitige Messoberfläche 13 ist dabei durchgängig eben ausgebildet und kann beispielsweise einen rechteckigen Umfang aufweisen.

Zur Bestimmung der Temperatur der Kondensationsoberfläche 12 ist im Bereich dieser Kondensationsoberfläche 12 auf dem transparenten Körper 10 ein schichtartig ausgebildeter, temperaturabhängiger Leiter 52 aufgebracht. Mittels dieses temperaturabhängigen Leiters 52, der die Kondensationsoberfläche 12 in Draufsicht vorteilhafterweise möglichst vollständig umfängt, kann die Temperatur der Kondensationsoberfläche 12 mit geringem Wärmeleitungskoeffizienten erfasst werden.

Die Ausnehmung 74 in der Reflexionsschicht 73 ist so positioniert, dass das aus der Lichtquelle 31 in den Lichtleiter 10 austretende Lichtbündel 1 schräg auf die Kondensationsoberfläche 12 trifft.

Vorzugsweise trifft das Lichtbündel 1 unter einem Einfallswinkel α auf die Kondensationsoberfläche 12, der zwischen dem Grenzwinkel α_{G} des Übergangs Lichtleiter/Messgas und dem Grenzwinkel α_{G} des Übergangs Lichtleiters/kondensierende Phase liegt. Ferner ist es vorteilhaft, wenn der Einfallswinkel α größer ist als der Grenzwinkel α_{G} des Übergangs kondensierende Phase/Messgas.

Fig. 1 zeigt den Sensor mit betauter Kondensationsoberfläche 12, wenn also die Oberflächentemperatur der Kondensationsoberfläche 12 unterhalb des Taupunktes des Messgases liegt. Bei diesen Temperaturverhältnissen ist auf der Kondensationsoberfläche 12 ein ausgedehnter Kondensatfilm 6 ausgebildet. Bei geeignet gewähltem Einfallswinkel α tritt zwar durch die aufgeraute Kondensationsoberfläche 12 Lichtintensität aus dem transparenten Körper 10 in den Kondensatfilm 6 aus. Die ausgekoppelte Lichtintensität wird jedoch an der Grenzfläche zwischen dem Kondensatfilm 6 und dem umgebenden Gas wieder zurückreflektiert und gelangt somit zumindest teilweise in den transparenten Körper 10 zurück.

Zumindest auf dem der Kondensationsoberfläche 12 gegenüberliegenden Rückseitenabschnitt ist die Rückseite 19 des transparenten Körpers 10 zur Verbesserung der Lichtreflexion mit einer Reflexionsschicht 46 versehen. Das auf die Kondensationsoberfläche 12 einfallende Lichtbündel 1 wird mehrfach zwischen der Kondensationsoberfläche 12, d.h. der Grenzfläche des darauf befindlichen Kondensatfilms 6, und der gegenüberliegenden Reflexionsschicht 46 hin- und herreflektiert. Nach einer abschließenden Reflexion an der Kondensationsoberfläche 12 tritt es durch eine eine weitere Blende bildende Ausnehmung 79 in einer weiteren Reflexionsschicht 78 durch eine weitere Immersionsschicht 76 auf eine sensitive Schicht 35 eines Lichtdetektors 34, der ebenfalls an der Rückseite 19 des transparenten Körpers 10 angeordnet ist.

Steigt nun die Temperatur der Kondensationsoberfläche 12 über die Taupunkttemperatur, so verdampft der Kondensatfilm 6. Infolgedessen ändern sich auch die optischen Verhältnisse an der Kondensationsoberfläche 12. Da nun keine Grenzfläche flüssig/gasförmig mehr gegeben ist, wird Licht, welches an der aufgerauten Kondensationsoberfläche 12 aus dem transparenten Körper 10 ausgekoppelt ist, nicht mehr in diesen transparenten Körper 10 zurückreflektiert, sondern verlässt diesen dauerhaft. Infolgedessen nimmt die Lichtintensität, die in den Lichtdetektor 34 gelangt, beim Überschreiten der Taupunkttemperatur ab.

Umgekehrt nimmt die vom Lichtdetektor 34 registrierte Lichtintensität wieder zu, wenn die Kondensationsoberfläche 12 erneut unter die Taupunkttemperatur abgekühlt wird, und sich ein Kondensatfilm 6 bildet. Ein Absinken der Lichtintensität am Lichtdetektor 34 bei steigender Temperatur und ein Ansteigen der Lichtintensität am Lichtdetektor 34 bei abnehmender Temperatur lassen somit.erkennen, dass der Taupunkt überschritten beziehungsweise unterschritten wurde.

Der in Fig. 1 dargestellte Sensor weist ein Gehäuse 20 auf, in dem der transparente Körper 10, die Lichtquelle 31, der Lichtdetektor 34 und das Peltier-Element 42 aufgenommen sind. Das Gehäuse 20 ist im Bereich der Kondensationsoberfläche 12 mit einer Öffnung 21 versehen, um die Wechselwirkung des Messgases mit der Kondensationsoberfläche 12 zu gewährleisten. Auf seiner Rückseite weist das Gehäuse 20 einen Boden 22 auf, an dem das Peltier-Element Wärme leitend angekoppelt ist. Am Boden 22 sind Befestigungsmittel 49 zum Befestigen einer in Fig. 1 nicht dargestellten Wärmesenke vorgesehen. Zur Kontaktierung der Lichtquelle 31, des Lichtdetektors 34, des Peltier-Elementes 42 sowie des temperaturabhängigen Leiters 52 sind am Boden 22 des Gehäuses 20 elektrische Kontaktstifte 48 vorgesehen.

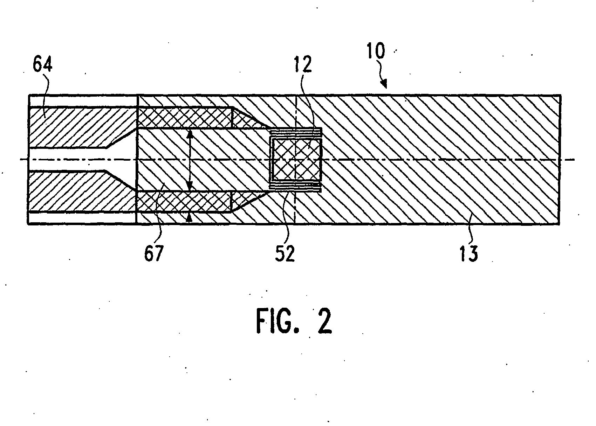

Ein weiteres Ausführungsbeispiel eines erfindungsgemäßen Sensors ist in Draufsicht in Fig. 2 dargestellt. Fig. 2 zeigt, dass auf einer ansonsten mikroskopisch glatten Messoberfläche 13 eine aufgeraute Kondensationsoberfläche 12 mit z.B. rechteckigem Außenumfang vorgesehen ist. Zur Bestimmung der Temperatur der Kondensationsoberfläche 12 ist diese auf der Messoberfläche 13 von einem temperaturabhängigen Leiter 52 umgeben, der die Kondensationsoberfläche 12 z.B. U-förmig umfasst. Zur Verbesserung der Genauigkeit der Temperaturbestimmung verläuft der temperaturabhängige Leiter 52 beiderseits der Kondensationsoberfläche 12 mehrfach mäanderartig entlang der Längsseite der Kondensationsoberfläche 12 vor und zurück. Zum Kontaktieren des temperaturabhängigen Leiters 52 sind auf der Messoberfläche 13 zwei Kupferkontakte 64 vorgesehen. Diese Kontakte 64 können beispielsweise auch Gold oder Silber aufweisen. Im Bereich der Kondensationsoberfläche 12 und des temperaturabhängigen Leiters 52 ist auf die Messoberfläche 13 ferner eine Passivierungsschicht 67 aufgebracht. Die Kondensationsoberfläche 12 kann auch größere Bereiche der Messoberfläche 13 bedecken.

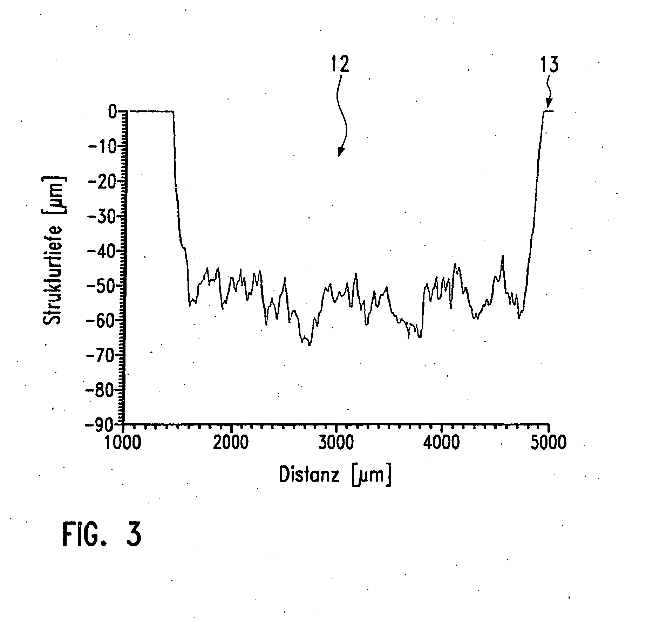

Fig. 3 zeigt ein beispielhaftes Oberflächenprofil des transparenten Körpers eines erfindungsgemäßen Sensors. Während die Kondensationsoberfläche 12 im Bereich der Messoberfläche 13 eine mikroskopische Rauigkeit im µm-Bereich aufweist, ist die umgebende Messoberfläche 13 mikroskopisch glatt.

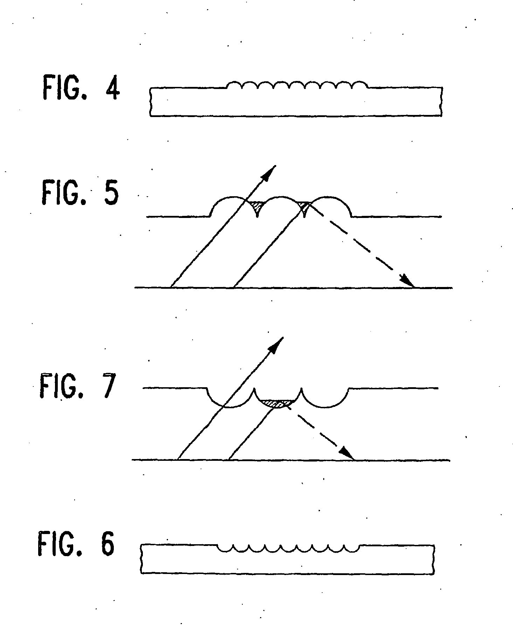

Verschiedene Ausführungsbeispiele von erfindungsgemäßen Kondensationsoberflächen sind in den Figuren 4 bis 12 dargestellt. Bei den in diesen Figuren dargestellten Oberflächen handelt es sich um strukturierte Oberflächen, die mittels Mikrostrukturtechniken hergestellt sind und bei denen die Oberflächenstrukturen nicht statistisch angeordnet sind. In diesem Fall können auf der Kondensationsoberfläche "künstliche" Wassertropfen und/oder Eiskristalle vorgesehen werden.

Bei dem Ausführungsbeispiel der Figuren 4 und 5 sind im Querschnitt konvexe, rundliche Vorsprünge auf der Messoberfläche vorgesehen. Der resultierende Strahlengang bei Betauung ist schematisch in Fig. 5 dargestellt.

Gemäß Figuren 6 und 7 sind konkave Vertiefungen auf der Kondensationsoberfläche vorgesehen. Der resultierende Strahlengang bei teilweise Betauung ist in Fig. 7 dargestellt.

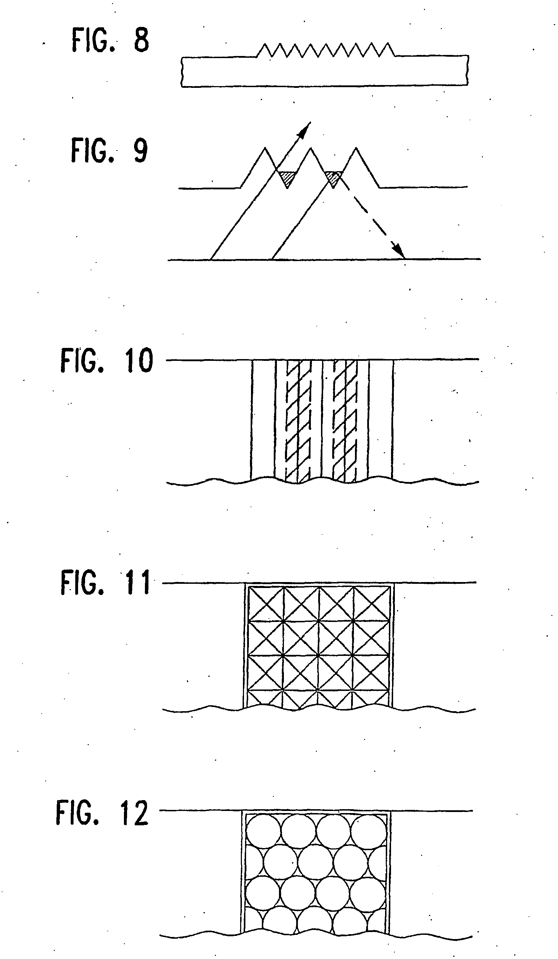

Die konvexen Erhöhungen der Figuren 4 und 5 und/oder die konkaven Vertiefungen der Figuren 6 und 7 können als parallel verlaufende Rillen ausgebildet sein. Sie können aber auch als kugelsegmentartige Erhöhungen oder Vertiefungen ausgebildet sein, wie in der Draufsicht der Fig. 12 gezeigt ist.

Gemäß dem Ausführungsbeispiel der Figuren 8 und 9 sind im Querschnitt dreieckige Erhöhungen vorgesehen. Der resultierende Strahlengang ist schematisch in Fig. 9 dargestellt.

Wie in Fig. 10 dargestellt ist, können die dreieckigen Erhöhungen parallel verlaufende Rillen bilden. Wie in Fig. 11 dargestellt ist, können diese aber auch beispielsweise pyramidenartige Strukturen bilden.

## Patentansprüche

1. Verfahren zum Bestimmen des Taupunktes in einem Gas, insbesondere in einem gasförmigen Brennstoff, mittels eines Sensors mit einem transparenten Körper (10), der eine planare Messoberfläche (13) aufweist, auf der eine Kondensationsoberfläche (12) mit kondensationsabhängigem Reflexionsvermögen vorgesehen ist, bei dem
- die Kondensationsoberfläche (12) mit dem Gas beaufschlagt wird,
- die Kondensationsoberfläche (12) durch den transparenten Körper (10) hindurch mit Licht bestrahlt wird,
- die Temperatur der Kondensationsoberfläche (12) verändert wird, und
- die von der Kondensationsoberfläche (12) in den transparenten Körper (10) zurückreflektierte Lichtintensität bestimmt wird,
**dadurch gekenunzeichnet,**
- **dass** der Taupunkt der Kohlenwasserstoff-Taupunkt ist,
- dass die Kondensationsoberfläche (12) aufgeraut ausgebildet wird, und
- dass beim Auftreten einer Intensitätszunahme des zurückreflektierten Lichtes bei abnehmender Temperatur und/oder beim Auftreten einer Intensitätsabnahme des zurückreflektierten Lichtes bei steigender Temperatur der Kondensationsoberfläche (12) die aktuelle Temperatur der Kondensationsoberfläche (12) bestimmt wird und als Maß für die Taupunkttemperatur ausgegeben wird.

2. Sensor zum Bestimmen des Taupunktes in einem Gas, bevorzugt in.einem gasförmigen Brennstoff, insbesondere zur Durchführung des Verfahrens nach Anspruch 1, mit
- einem transparenten Körper (10) mit einer planaren Messoberfläche (13), auf der eine Kondensationsoberfläche (12) mit kondensationsabhängigem Reflexionsvermögen angeordnet ist,
- einer Lichtquelle (31) zum Aussenden von Licht durch den transparenten Körper (10) auf die Kondensationsoberfläche (12),
- einem Lichtdetektor (34) zum Ermitteln der von der Kondensationsoberfläche (12) in den transparenten Körper (10) zurückreflektierten Lichtintensität,
- Mitteln zum Einstellen der Temperatur der Kondensationsoberfläche (12), und
- einer Auswerteeinheit, die mit dem Lichtdetektor (34) in Signalverbindung steht,
**dadurch gekennzeichnet,**
- **dass** der Taupunkt der Kohlenwasserstoff-Taupunkt ist,
- **dass** die Kondensationsoberfläche (12) aufgeraut ist, und
- **dass** die Auswerteeinheit dazu eingerichtet ist, beim Auftreten einer Intensitätszunahme des zurückreflektierten Lichtes bei abnehmender Temperatur und/oder einer Intensitätsabnahme des zurückreflektierten Lichtes bei steigender Temperatur der Kondensationsoberfläche (12) die entsprechende Temperatur der Kondensationsoberfläche (12) zu bestimmen und als Maß für die Taupunkttemperatur auszugeben.

3. Sensor nach Anspruch 2,
**dadurch gekennzeichnet,**
**dass** er eine Einrichtung zum Messen der Temperatur der Kondensationsoberfläche (12) aufweist, die mit der Auswerteeinheit in Signalverbindung steht.

4. Sensor nach einem der Ansprüche 2 oder 3,
**dadurch gekennzeichnet,**
**dass** der transparente Körper (10) ein Glaskörper, insbesondere ein Borosilikat-Glaskörper, bevorzugt ein Borofloat-Glaskörper ist.

5. Sensor nach einem der Ansprüche 2 bis 4,
**dadurch gekennzeichnet,**
**dass** die aufgeraute Kondensationsoberfläche (12) eine Glasoberfläche oder eine beschichtete Glasoberfläche aufweist.

6. Sensor nach einem der Ansprüche 2 bis 5,
**dadurch gekennzeichnet,**
**dass** die Kondensationsoberfläche (12) eine Wasser und/oder Schmutz abweisende Beschichtung aufweist.

7. Sensor nach einem der Ansprüche 2 bis 6,
**dadurch gekennzeichnet,**
**dass** die Kondensationsoberfläche (12) lipophil ausgebildet ist.

8. Sensor nach einem der Ansprüche 2 bis 7,
**dadurch gekennzeichnet,**
**dass** die Kondensationsoberfläche (12) eine gemittelte Rautiefe zwischen 1 µm und 100 µm, insbesondere von etwa 10 µm, aufweist.

9. Sensor nach einem der Ansprüche 2 bis 8,
**dadurch gekennzeichnet,**
**dass** die Messoberfläche (13) in der Umgebung der Kondensationsoberfläche (12) ebenfalls aufgeraut ist.

10. Sensor nach einem der Ansprüche 2 bis 9,
**dadurch gekennzeichnet,**
**dass** der transparente Körper (10) eine der planaren Messoberfläche (13) abgewandte Rückseitenfläche aufweist, die zumindest bereichsweise parallel zur Messoberfläche (13) verläuft, wobei die Lichtquelle (31) und/oder der Lichtdetektor (34) in den parallel verlaufenden Bereichen der Rückseitenfläche angeordnet sind.

11. Sensor nach einem der Ansprüche 2 bis 10,
**dadurch gekennzeichnet,**
**dass** die Lichtquelle (31) und/oder der Lichtdetektor (34) bezüglich der Kondensationsoberfläche (12) seitlich versetzt angeordnet sind.

12. Sensor nach einem der Ansprüche 2 bis 11,
**dadurch gekennzeichnet,**
**dass** die Mittel zum Einstellen der Temperatur der Kondensationsoberfläche (12) ein, vorzugsweise mehrstufiges, Peltier-Element (42), aufweisen.

13. Sensor nach einem der Ansprüche 2 bis 12,
**dadurch gekennzeichnet,**
**dass** er eine Druckerzeugungseinrichtung aufweist, durch welche das Gas an der Kondensationsoberfläche (12) mit Druck beaufschlagbar ist.

14. Verwendung des Sensors nach einem der Ansprüche 2 bis 13 zur Bestimmung der Taupunkttemperatur von Kohlenwasserstoffen, insbesondere gasförmigen Brennstoffen.

## Claims

1. Method for determining the dew point in a gas, particularly in a gaseous fuel, by means of a sensor having a transparent body (10) which has a planar measurement surface (13) on which is provided a condensation surface (12) with a condensation-dependent reflectivity, in which
- the gas is applied to the condensation surface (12),
- through the transparent body (10) the condensation surface (12) is irradiated with light,
- the temperature of the condensation surface (12) is changed and,
- the light intensity reflected back into the transparent body (10) from the condensation surface (12) is determined,
**characterized in that**
- the dew point is the hydrocarbon dew point,
- the condensation surface (12) is roughened, and
- when there is an intensity increase of the re-reflected light at a decreasing temperature and/or an intensity decrease of the re-reflected light at a rising temperature of the condensation surface (12), the present temperature of the condensation surface (12) is determined and is output as a measure for the dew point temperature.

2. Sensor for determining the dew point in a gas, preferably in a gaseous fuel, particularly for performing the method according to claim 1, having
- a transparent body (10) with a planar measurement surface (13) on which is placed a condensation surface (12) with a condensation-dependent reflectivity,
- a light source (31) for emitting light onto the condensation surface (12) through the transparent body (10),
- a light detector (34) for determining the light intensity re-reflected into the transparent body (10) by the condensation surface (12),
- means for adjusting the temperature of the condensation surface (12), and
- an evaluating unit in signal connection with the light detector (34), **characterized in that**
- the dew point is the hydrocarbon dew point,
- the condensation surface (12) is roughened, and
- the evaluating unit is set up so that when there is an intensity increase of the re-reflected light at a decreasing temperature and/or an intensity decrease of the re-reflected light at a rising temperature of the condensation surface (12), the corresponding temperature of the condensation surface (12) is determined and output as a measure for the dew point temperature.

3. Sensor according to claim 2,
**characterized in that**
it has a device for measuring the temperature of the condensation surface (12), which is in signal connection with the evaluating unit.

4. Sensor according to one of the claims 2 or 3,
**characterized in that**
the transparent body (10) is a glass body, particularly a borosilicate glass body, preferably a borofloat glass body.

5. Sensor according to one of the claims 2 to 4,
**characterized in that**
the roughened condensation surface (12) has a glass surface or a coated glass surface.

6. Sensor according to one of the claims 2 to 5,
**characterized in that**
the condensation surface (12) has a water- and/or dirt-repelling coating.

7. Sensor according to one of the claims 2 to 6,
**characterized in that**
the condensation surface (12) has a lipophilic structure.

8. Sensor according to one of the claims 2 to 7,
**characterized in that**
the condensation surface (12) has an averaged peak-to-valley height of between 1 µm and 100 µm, particularly of approximately 10 µm.

9. Sensor according to one of the claims 2 to 8,
**characterized in that,**
in the vicinity of the condensation surface (12), the measurement surface (13) is also roughened.

10. Sensor according to one of the claims 2 to 9,
**characterized in that**
the transparent body (10) has a back surface facing away from the planar measurement surface (13), which runs at least in some areas parallel to the measurement surface (13), the light source (31) and/or light detector (34) being located in the parallel running areas of the back surface.

11. Sensor according to one of the claims 2 to 10,
**characterized in that,**
with respect to the condensation surface (12), the light source (31) and/or light detector (34) are located laterally displaced.

12. Sensor according to one of the claims 2 to 11,
**characterized in that**
the means for adjusting the temperature of the condensation surface (12) have a preferably multistage Peltier element (42).

13. Sensor according to one of the claims 2 to 12,
**characterized in that**
it has a pressure generating device through which the gas at the condensation surface (12) can be pressurized.

14. Use of the sensor according to one of the claims 2 to 13 for determining the dew point temperature of hydrocarbons, particularly gaseous fuels.

## Revendications

1. Procédé pour déterminer le point de rosée dans un gaz, en particulier dans un combustible gazeux, au moyen d'un capteur avec un corps transparent (10), qui présente une surface de mesure plane (13), sur laquelle est prévue une surface de condensation (12) à pouvoir de réflexion fonction de la condensation, dans lequel
- la surface de condensation (12) est exposée au gaz,
- la surface de condensation (12) est exposée à de la lumière à travers le corps transparent (10),
- la température de la surface de condensation (12) est modifiée, et
- l'intensité lumineuse réfléchie dans le corps transparent (10) par la surface de condensation (12) est déterminée,
***caractérisé***
- ***en ce que*** le point de rosée est le point de rosée d'hydrocarbure,
- ***en ce que*** la surface de condensation (12) est réalisée rugueuse, et
- ***en ce que**,* en présence d'une augmentation d'intensité de la lumière réfléchie pour une température décroissante et/ou en présence d'une réduction d'intensité de la lumière réfléchie pour une température croissante de la surface de condensation (12), la température actuelle de la surface de condensation (12) est déterminée et transmise comme mesure de la température du point de rosée.

2. Capteur pour déterminer le point de rosée dans un gaz, en particulier dans un combustible gazeux, en particulier pour mettre en oeuvre le procédé selon la revendication 1, avec
- un corps transparent (10) avec une surface de mesure plane (13), sur laquelle est placée une surface de condensation (12) à pouvoir réfléchissant fonction de la condensation,
- une source lumineuse (31) pour envoyer de la lumière, à travers le corps transparent (10), sur la surface de condensation (12),
- un détecteur de lumière (34) pour déterminer l'intensité lumineuse réfléchie par la surface de condensation (12) dans le corps transparent (10),
- des moyens pour régler la température de la surface de condensation (12), et
- une unité d'analyse qui est en liaison de signal avec le détecteur de lumière (34), ***caractérisé***
- ***en* ce *que*** le point de rosée est le point de rosée d'hydrocarbure,
- ***en* ce *que*** la surface de condensation (12) est réalisée rugueuse, et
- ***en ce que*** l'unité d'analyse est agencée pour, en présence d'une augmentation d'intensité de la lumière réfléchie pour une température décroissante et/ou en présence d'une réduction d'intensité de la lumière réfléchie pour une température croissante de la surface de condensation (12), déterminer la température correspondante de la surface de condensation (12) et la transmettre comme mesure de la température du point de rosée.

3. Capteur selon la revendication 2, ***caractérisé en ce qu*'**il comprend un dispositif pour mesurer la température de la surface de condensation (12), qui est en liaison de signal avec l'unité d'analyse.

4. Capteur selon l'une quelconque des revendications 2 ou 3, ***caractérisé en ce que*** le corps transparent (10) est un corps en verre, en particulier un corps en verre borosilicaté, de préférence un corps en verre boro-flotté.

5. Capteur selon l'une quelconque des revendications 2 à 4, ***caractérisé en ce que*** la surface de condensation rugueuse (12) comprend une surface en verre ou une surface en verre enduite.

6. Capteur selon l'une quelconque des revendications 2 à 5, ***caractérisé en ce que*** la surface de condensation (12) comprend un revêtement repoussant l'eau et/ou la saleté.

7. Capteur selon l'une quelconque des revendications 2 à 6, ***caractérisé en ce que*** la surface de condensation (12) est réalisée lipophile.

8. Capteur selon l'une quelconque des revendications 2 à 7, ***caractérisé en* ce *que*** la surface de condensation (12) présente une rugosité comprise en 1 µm et 100 µm, en particulier d'environ 10 µm.

9. Capteur selon l'une quelconque des revendications 2 à 8, ***caractérisé en ce que*** la surface de mesure (13) est également rugueuse au voisinage de la surface de condensation (12).

10. Capteur selon l'une quelconque des revendications 2 à 9, ***caractérisé en ce que*** le corps transparent (10) comprend une surface latérale arrière opposée à la surface de mesure plane (13), qui s'étend au moins par endroits parallèlement à la surface de mesure (13), la source lumineuse (31) et/ou le détecteur de lumière (34) étant placés dans les zones parallèles de la surface latérale arrière.

11. Capteur selon l'une quelconque des revendications 2 à 10, ***caractérisé* en ce *que*** la source lumineuse (31) et/ou le détecteur de lumière (34) sont décalés latéralement par rapport à la surface de condensation (12).

12. Capteur selon l'une quelconque des revendications 2 à 11, ***caractérisé en ce que*** les moyens de réglage de la température de la surface de condensation (12) comprennent un élément à effet Peltier (42), de préférence à plusieurs niveaux.

13. Capteur selon l'une quelconque des revendications 2 à 12, ***caractérisé en ce qu*'**il comprend un dispositif de production de pression, par lequel le gaz peut être envoyé sous pression sur la surface de condensation (12).

14. Utilisation du capteur selon l'une quelconque des revendications 2 à 13 pour déterminer la température du point de rosée d'hydrocarbures, en particulier de combustibles gazeux.
